# DEMANDE DE BREVET EUROPEEN

(11) **EP 4 843 521 A1**
(43) Date de publication de la demande: **25.11.2026**
(21) Numéro de dépôt: 26180131.0
(22) Date de dépôt: 21.05.2026
(51) Int. Cl.: A61B 17/3211, A61B 17/3213, A61B 90/00

(54) **INSTRUMENT POUR CHIRURGIE BUCCALE**

(30) Priorité: 21.05.2025 FR 2505451
(71) Demandeur: The Bone Institute, 06000 Nice (FR)
(72) Inventeur: SURMENIAN, Jérôme, 06000 Nice (FR)
(74) Mandataire: Barbot, Willy

(57) **Abrégé**

Un instrument pour chirurgie buccale comprenant un manche, une zone d'assemblage sur laquelle peut être fixée une lame, et une butée, caractérisé en ce que la butée, une fois la lame fixée, est disposée à un écartement prédéfini et constant du au moins un bord tranchant de la lame sur toute son étendue.

## Description

La présente demande de brevet revendique la priorité de la demande de brevet Français FR 2505451 déposée en date du 21 mai 2025.

### Domaine de l'invention

La présente invention concerne un instrument pour chirurgie buccale et, plus spécifiquement un instrument pour chirurgie buccale présentant une butée adaptée aux interventions de chirurgie buccale.

### Etat de l'art

Depuis toujours, les scalpels accompagnent la main du chirurgien avec l'objectif constant de lui assurer un geste à la fois sûr et précis.

Dans ce cadre, la chirurgie impose des incisions maîtrisées et régulières pour prévenir les cicatrices. C'est notamment le cas en chirurgie buccale où le champ opératoire est étroit et les structures anatomiques délicates, notamment le périoste qui se trouve sous la gencive. Ce périoste est en fait une membrane fibreuse très fine (0,4mm à 0,6mm) qui enveloppe l'os et assure sa vascularisation ainsi que sa régénération. Lors de l'ouverture de la gencive, avec la réalisation d'un lambeau muco-périosté, l'objectif est de traverser la muqueuse avec précision pour atteindre et inciser uniquement le périoste. En effet, une incision trop profonde pourrait léser le tissu sous-jacent au périoste et compromettre la cicatrisation. À ce jour, la gestion de ce risque repose entièrement sur la précision et la dextérité du chirurgien.

Pour répondre à cette problématique, certains instruments d'incision ont été conçus avec un guide de profondeur afin de mieux contrôler la précision du geste chirurgical. Le brevet EP 0 236 485 B1 en est un exemple, lequel propose une solution visant à garantir une profondeur d'incision plus contrôlée. La demande de brevet CN 107 811 682 A en est un autre exemple et décrit un scalpel avec une pièce supportant la lame et une pièce complémentaire formant une butée définissant la profondeur de coupe, laquelle profondeur est réglable au moyen d'une molette réglable. La demande de brevet US 2004/087989 A1 décrit un scalpel comprenant un manche, une lame et une gaine traversée par la lame. Dans cet exemple, la gaine peut recouvrir la lame ou, lorsqu'elle avance, exposer le bord tranchant de 2 à 5 mm tout en gardant la pointe couverte. Enfin, la demande de brevet CN 112 890 989 A décrit un ostéotome creux destiné à la chirurgie bucco-dentaire, intégrant un positionnement réglable et une visualisation per-opératoire. Le dispositif décrit comprend un corps de lame muni d'un manchon. Du côté lame, le manchon porte une microcaméra reliée par un fil à un écran externe, tandis qu'à l'extrémité opposée il est soudé à un bloc de limitation. Le positionnement est obtenu par rotation d'un bouton de réglage qui actionne une vis et déplace une colonne télescopique le long d'un rail de guidage.

Toutefois, ces systèmes demeurent encombrants ou incompatibles avec les lames standardisées, notamment celles répondant à la norme ISO 7740. De plus, ils peinent à garantir une profondeur d'incision réellement constante. Ces limites deviennent particulièrement problématiques en chirurgie buccale où l'espace restreint, la visibilité réduite et l'exigence de précision rendent leur utilisation difficile.

Par ailleurs, la standardisation accrue des instruments (lames jetables, manches réutilisables) répond à des impératifs économiques, écologiques et de traçabilité, qui impose désormais le développement de solutions compatibles. Celles-ci doivent impérativement respecter les normes internationales (ISO, ANSI/AAMI) et s'intégrer, entre autres, aux protocoles de stérilisation en vigueur.

Malgré ces avancées, la régulation passive, fiable et homogène de la profondeur d'incision demeure un enjeu non résolu en chirurgie buccale. Les stomatologues et les parodontistes qui accordent une importance majeure à la précision submillimétrique, essentielle pour une bonne cicatrisation, continuent donc à exprimer un réel besoin pour un instrument qui assure à la fois une régularité du geste, une sécurité d'utilisation et une compatibilité avec les standards en vigueur.

### Sommaire de l'invention

Face à ce besoin persistant d'un contrôle passif, reproductible et sécurisé de la profondeur d'incision, la présente invention propose un instrument pour chirurgie buccale permettant de limiter simplement et mécaniquement la profondeur d'incision de la lame, tout en restant compatible avec les standards existants.

En conséquence, un premier objet selon l'invention consiste en un instrument (7) pour chirurgie buccale comprenant 1) un manche (3) et 2) une extrémité active comprenant :
i) une zone d'assemblage (1) présentant au moins un segment de liaison conçu pour recevoir et fixer une lame (4) détachable sur une face,
ii) une butée (6) associée à ladite zone d'assemblage, sur sa face opposée à celle conçue pour recevoir et fixer la lame (4) détachable, et
iii) éventuellement, une lame (4) détachable fixée sur le segment de liaison de ladite zone d'assemblage (1),
   où lorsque la lame (4) détachable est fixée sur le segment de liaison de ladite zone d'assemblage (1), son bord tranchant (5) fait saillie au-delà de la butée (6) pour définir une profondeur de coupe, laquelle butée (6) présente un bord, le plus proche dudit bord tranchant (5), qui :
   - présente une longueur supérieure ou égale à celle dudit bord tranchant (5),
   - est parallèle audit bord tranchant (5) sur au moins toute la longueur dudit bord tranchant (5), et
   - présente un écartement (D) prédéterminé, de préférence constant, avec ledit bord tranchant (5) de sorte à définir une profondeur de coupe maximale (cette profondeur de coupe maximale correspondant audit écartement (D)).

Cette configuration permet de limiter précisément la profondeur d'incision, quel que soit le segment de lame utilisée, et donc de renforcer la sécurité en chirurgie buccale. En effet, la butée, une fois la lame fixée, est disposée à un écartement constant du au moins un bord tranchant de la lame sur toute son étendue.

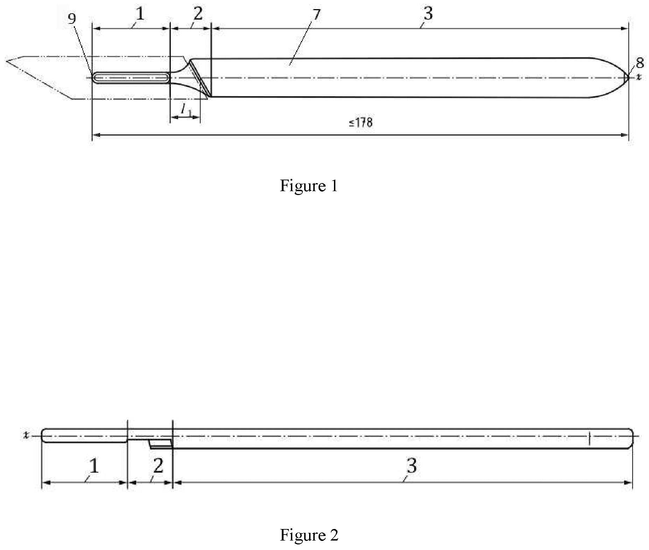

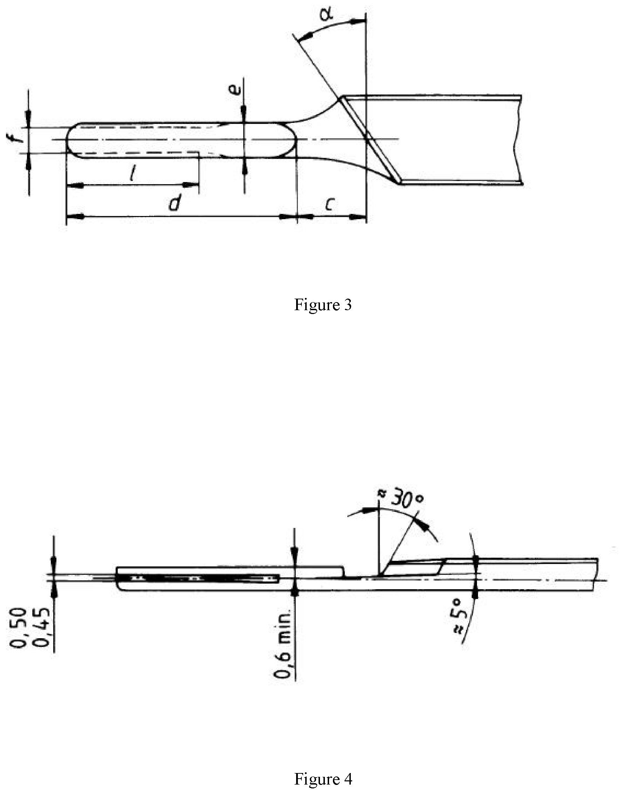

La demande de brevet chinois CN 107 811 682 A décrit un scalpel à profondeur de coupe réglable. Le dispositif comporte deux pièces distinctes. D'une part, une pièce supporte la lame comme montré à la figure 3. D'autre part, une pièce forme la butée de limitation comme montré à la figure 2, de sorte que la profondeur correspond à la longueur de lame qui dépasse. Ainsi, la profondeur se règle par vissage au moyen d'un écrou sur un boulon gradué. Puis le réglage est bloqué par un écrou de verrouillage. L'échelle graduée indique directement la profondeur choisie, laquelle n'a cependant rien de constant mais varie en fonction de la section de bord tranchant utilisé.

Dans le cas d'une profondeur de coupe limitée à moins d'un millimètre, il devient possible de limiter l'incision au seul périoste, dont l'épaisseur moyenne est de l'ordre de 0,5mm, et ceci en évitant de léser l'os, les nerfs ou les vaisseaux sous-jacents et donc de prévenir la perforation des lambeaux tissulaires. Idéalement, l'écartement (D) est de 0,5 mm ± 0,05 mm. Ainsi, cette profondeur de coupe limitée permet même de préserver la couche interne du périoste, le cambium, essentielle à la régénération osseuse.

L'écartement (D) prédéterminé entre le bord tranchant (5) de la lame (4) détachable et le bord parallèle de la butée (6) est défini comme la distance constante, sur toute l'étendue du bord tranchant, entre ce bord tranchant et le bord parallèle de la butée, avec une variation tolérée inférieure ou égale à ± 0,05 mm.

Avantageusement, la butée est partie intégrante de la zone d'assemblage (1) qui ne forme alors qu'une seule et même pièce

Selon un autre mode de réalisation préféré, la zone d'assemblage comprend en outre une surface d'appui sur laquelle se positionne la lame détachable une fois fixée par le segment de liaison, laquelle surface d'appui ne couvre pas le bord tranchant de la lame détachable.

Cette surface d'appui présente l'avantage de contribuer, avec le segment de fixation, à la fixation de la lame détachable, du fait du contact entre elle et la lame détachable, lequel contact contribue à augmenter la résistance à l'arrachement/mouvement de la lame détachable une fois en place dans le segment de fixation.

De préférence, cette fonction de surface d'appui est assurée, pour tout ou partie, par la butée.

De préférence, la forme de cette surface d'appui est telle qu'elle permet, à un opérateur, d'accéder à au moins deux segments opposés de la lame détachable, dont aucun n'intègre tout ou partie de son bord tranchant et dont la longueur est d'au moins un centimètre, de préférence d'au moins 1,5 centimètres. Une telle dimension vise à permettre leur accessibilité à des doigts humains lors de la mise en place ou du retrait de la lame détachable par l'opérateur.

L'instrument (7) pour chirurgie buccale présente, de préférence, un segment de liaison présentant une configuration du type « petite fenêtre » selon la norme ISO7740.

Une telle configuration est avantageuse car elle accepte directement les lames détachables 15 et 15C, idéales pour les coupes fines et courbes en chirurgie buccale.

De préférence, les dimensions de la butée et de la zone d'assemblage (longueur, largeur, épaisseur) respectent des tolérances strictes pour garantir la compatibilité avec les lames détachables usuelles (par exemple de type 15 ou 15C).

Selon un mode de réalisation préféré complémentaire, la butée est partie intégrante de la zone d'assemblage qui ne forme alors qu'une seule et même pièce.

### Description des figures

La figure 1 est une représentation de côté d'un instrument pour chirurgie buccale classique
La figure 2 est une représentation de dessus du même instrument pour chirurgie buccale classique.
La figure 3 est une représentation de côté de l'extrémité active du même instrument pour chirurgie buccale classique.
La figure 4 est une représentation de dessus de l'extrémité active du même instrument pour chirurgie buccale classique.
La figure 5 est une représentation de côté de l'extrémité active du même instrument pour chirurgie buccale classique et où la zone d'assemblage présente une lame détachable fixée.
La figure 6 est une représentation de côté d'un instrument pour chirurgie buccale selon l'invention.
La figure 7 est une représentation de dessus de l'extrémité active du même instrument pour chirurgie buccale selon l'invention.
La figure 8 est une représentation de côté de l'extrémité active du même instrument pour chirurgie buccale selon l'invention classique et où la zone d'assemblage présente une lame détachable fixée.

### Description détaillée

Dans le cadre de la présente demande, les termes « instrument pour chirurgie buccale », « scalpel chirurgical » ou encore « bistouri » désignent indifféremment un instrument chirurgical pouvant comprendre une lame tranchante destinée à inciser.

L'instrument selon l'invention comprend un manche et une extrémité active.

Conformément à la norme ISO 20570, un tel instrument comprend un point proximal et un point distal.

Par « point distal », on entend le point le plus proche du bord tranchant de la lame détachable. Plus généralement, l'expression « côté distal », en référence à l'instrument selon l'invention ou à l'un de ses éléments, se réfèrera au côté le plus proche du bord tranchant de la lame détachable.

Par « point proximal », on entend le point le plus éloigné du bord tranchant de la lame détachable. Plus généralement, l'expression « côté proximal », en référence à l'instrument selon l'invention ou à l'un de ses éléments, se réfèrera au côté le plus éloigné du bord tranchant de la lame détachable.

Un « manche », conformément à la norme ISO 20570, désigne la partie d'un instrument permettant sa préhension lors de son utilisation.

Afin d'offrir une prise en main optimale, un tel manche présente une forme adaptée qui peut être cylindrique, tubulaire, hexagonale, ovale ou encore polygonale. De préférence et dans le prolongement de cette recherche d'ergonomie, le manche présente une surface améliorant sa préhension, laquelle pourra, à titre d'exemple, être rugueuse ou encore présenter des rainures. Les rainures renforcent l'adhérence et réduisent la fatigue de la main durant les gestes prolongés.

Selon un mode de réalisation préféré de l'invention et dans un souci de durabilité, la dureté VICKERS du manche est supérieure à 150 HV (test Vickers), de préférence supérieure à 220 HV (test Vickers).

Typiquement, la longueur totale de l'instrument selon l'invention est inférieure ou égale à 230 mm sans la lame détachable et, de façon préférée, inférieure ou égale à 200 mm sans la lame. La longueur de l'instrument selon l'invention intégrant la lame détachable variera en fonction de la longueur de cette dernière. Typiquement, l'instrument selon l'invention avec sa lame détachable présentera une longueur allant jusqu'à 235 mm et, de façon préférée, jusqu'à 205 mm.

On entend par « extrémité active », la partie de l'instrument pour chirurgie buccale selon l'invention intégrant le point distal et comprenant la zone d'assemblage avec son segment de liaison et la butée.

Avantageusement, et pour optimiser la modularité de l'instrument selon l'invention, l'extrémité active comprend en outre une queue.

Une telle « queue » assure la connexion mécanique entre le manche et la zone d'assemblage. La fonction de cette queue est d'offrir un degré de liberté supplémentaire pour ajuster la configuration de l'instrument selon l'invention sans complexifier le manche. La queue permet notamment d'ajuster la distance, l'angle ou encore la configuration de l'instrument selon l'invention entre la zone d'assemblage et le manche.

L'instrument pour chirurgie buccale selon l'invention peut ainsi être de type droit, coudé ou encore contre-coudé.

On entend par « instrument pour chirurgie buccale de type droit » et selon la norme ISO 20570, un instrument qui ne présente aucune angulation entre l'axe de la zone d'assemblage et l'axe du manche.

On entend par « instrument pour chirurgie buccale de type coudé » et selon la norme ISO 20570, un instrument qui présente, entre l'axe de la zone d'assemblage et l'axe du manche, une angulation (α compris entre 0 et 90°) au niveau de laquelle le point proximal de la zone d'assemblage est relié à la queue de l'instrument selon l'invention. Typiquement, pour un instrument pour chirurgie buccale de type coudé, la longueur de la queue sera supérieure ou égale à 4,50 mm.

On entend par « instrument pour chirurgie buccale de type contre-coudé » et selon la norme ISO 20570, un instrument pour chirurgie buccale qui présente un décalage angulaire (β compris entre 20 et 90°) entre l'axe de la zone d'assemblage et l'axe du manche où le point distal de la zone d'assemblage est relié à la queue du manche de l'instrument selon l'invention. Typiquement, pour un instrument pour chirurgie buccale de type contre-coudé, la longueur de la queue est supérieure ou égale à 22,31 × cos β en millimètres.

Typiquement, la queue de l'instrument selon l'invention présente une longueur allant de 1 à 50 millimètres, de préférence de 2 à 30 millimètres et, de manière particulièrement préférée, de 4 à 25 millimètres.

Selon un mode de réalisation particulier, l'extrémité active est amovible. Elle est alors conçue pour être détachée du manche selon un axe de démontage prédéfini. Dans ce cas, le côté proximal de l'extrémité active (c'est-à-dire la partie de l'extrémité active destinée à être fixée au manche) et le côté distal du manche intègrent les parties complémentaires d'un système de fixation, de type vis avec un filetage (interne ou externe), de type emboîtement (conique ou forcé) comme un connecteur à détente à ressort (permettant un encliquetage rapide par mécanisme à came), intégrant un verrouillage, tel qu'un mécanisme par rotation ou par baïonnette, ou de type magnétique, notamment au moyen d'aimants en néodyme biocompatibles. Une telle configuration permet avec un seul et même manche et plusieurs extrémités actives distinctes de pouvoir bénéficier, selon le besoin, d'un instrument pour chirurgie buccale, qu'il soit de type droit, coudé ou encore contre-coudé.

La « zone d'assemblage » désigne la partie de l'instrument sur laquelle est fixée la lame détachable, laquelle est située au niveau de l'extrémité active de l'instrument selon l'invention. Pour cette fixation, la zone d'assemblage comprend donc au moins un segment de liaison dont la fonction est de solidariser la lame détachable à l'instrument selon l'invention. De façon générale, ledit au moins un segment de liaison permet le montage de la lame détachable sur l'instrument selon l'invention par coulissement.

De préférence, ce segment de liaison comprend un mécanisme anti-retour. Un tel mécanisme anti-retour permet de sécuriser la lame détachable une fois engagée.

Selon un autre mode de réalisation préféré, la zone d'assemblage comprend une surface d'appui sur laquelle se positionne la lame détachable une fois fixée par le segment de liaison, laquelle surface d'appui ne couvre pas le bord tranchant de la lame détachable.

Une telle surface d'appui peut, éventuellement, se trouver sur la queue de l'instrument selon l'invention si cette dernière est présente.

De façon générale, la surface d'appui, comme le segment de liaison, appartiennent à la zone d'assemblage, laquelle ne forme qu'une seule et même pièce.

De préférence, la forme de cette surface d'appui est telle qu'elle permet, à un opérateur, d'accéder à au moins deux segments opposés de la lame détachable, dont aucun n'intègre tout ou partie de son bord tranchant et dont la longueur est d'au moins un centimètre, de préférence d'au moins 1,5 centimètres.

Idéalement, ces au moins deux segments opposés sont localisés au niveau du côté proximal de la lame détachable.

On entend par « lame détachable », un élément muni d'au moins un bord tranchant destiné à inciser et qui peut être désolidarisé sans difficulté de l'instrument selon l'invention. Ce « bord tranchant » ou « arête de coupe » de la lame détachable correspond à l'arête formée par l'intersection des deux faces adjacentes de la lame détachable. Cette ligne, communément appelée « fil », constitue la partie active de coupe et, en fonction de la lame détachable utilisée, pourra être rectiligne ou courbée. En lien avec la solidarisation de la lame détachable sur la zone d'assemblage de l'instrument selon l'invention, cette lame détachable comporte une fenêtre d'assemblage, également appelée encoche ou encore orifice, dimensionnée selon la même norme ISO 7740. Lors de la mise en place de la lame détachable sur l'extrémité active de l'instrument selon l'invention, la lame détachable coulisse sur la zone d'assemblage et vient s'emboîter fermement, au niveau de sa fenêtre d'assemblage et sur le segment de liaison, assurant un alignement précis ainsi qu'un verrouillage de la lame détachable de façon sécurisée sur l'extrémité active.

De préférence, le segment de liaison est conforme à la norme ISO 7740 et est donc dimensionné de manière à assurer sa compatibilité avec des lames détachables standardisées.

La lame détachable peut ainsi intégrer une fenêtre d'assemblage n°3, dite « petite fenêtre d'assemblage », ou n°4, dite « grande fenêtre d'assemblage ». Chaque type de fenêtre d'assemblage est conçu pour accueillir une gamme spécifique de lames détachables.

Une petite fenêtre d'assemblage (détails des dimensions présentée dans le tableau 1), selon la norme ISO 7740, convient aux lames détachables de type 10, 11, 12, 15, 15C et 16. Ces lames détachables sont fines et adaptées aux interventions précises telles que l'ophtalmologie, la chirurgie plastique ou encore la chirurgie buccodentaire.

La grande fenêtre d'assemblage (détails des dimensions présentée dans le tableau 2) selon la norme ISO 7740, est destinée aux lames détachables de type 20, 21, 22, 23, 24 et 25, plus larges et recommandées pour les incisions profondes ou le travail sur des tissus denses, comme en orthopédie ou en chirurgie abdominale.

Aussi, et pour la chirurgie buccale, le segment de liaison de la zone d'assemblage de l'instrument selon l'invention est, de préférence, adapté à une lame détachable présentant une configuration « petite fenêtre » (n° 3 au sens de la norme ISO 7740). La configuration « petite fenêtre » s'avère alors particulièrement adaptée en raison de l'utilisation récurrente des lames détachables 15 et 15C dans ce contexte. De telles lames détachables sont bien connues de l'homme du métier et leurs dimensions sont notamment standardisées dans ANSI/AAMI MD 89 (Scalpels and Scalpel Blades - Specifications).

Typiquement, et selon la norme ISO 7740, pour un assemblage de type « petite fenêtre d'assemblage » (n° 3), l'épaisseur du segment de liaison de la zone d'assemblage est comprise entre 1,00 mm et 1,20 mm, de préférence entre 1,12 mm et 1,17 mm.

Selon l'invention, on entend par « butée » un élément mécanique faisant saillie, caractérisé par une proéminence structurale. Une telle butée agira, par le biais de son bord le plus proche du bord tranchant de la lame détachable et parallèle à ce dernier, comme un limiteur physique et permettra ainsi de maintenir une profondeur d'incision prédéfinie.

La butée, selon l'invention, est située du côté distal de la zone d'assemblage de l'instrument selon l'invention et pourra prendre la forme d'une plaque, d'une nervure d'arrêt, ou d'une tige rigide présentant un bord parallèle au bord tranchant, de préférence la butée prendra la forme d'une plaque. Le bord de la butée parallèle au bord tranchant de la lame détachable pourra, selon que ce bord tranchant soit rectiligne ou courbé, être lui aussi rectiligne ou courbé.

Avantageusement, et dans le cas où la zone d'assemblage comprend une surface d'appui, cette fonction (de surface d'appui) est assurée, pour tout ou partie, par la butée. Elle s'étend alors du côté proximal au côté distal de la lame détachable, en laissant le bord tranchant dégagé selon un écartement (D) prédéterminé.

Selon un mode de réalisation, la butée assure une fonction de surface d'appui sur au moins 50 % de la surface de la lame détachable, de préférence sur au moins 60 %, et, de manière particulièrement préférée, sur au moins 70 %.

Une telle configuration permet d'assurer un appui stable et étendu de la lame sur la butée, contribuant à la précision du geste chirurgical.

La butée peut être solidaire de l'extrémité active ou amovible.

Selon un mode de réalisation alternatif, la butée est amovible. Typiquement, et lorsqu'elle est amovible, la butée est fixée à l'instrument selon l'invention par assemblage mécanique, lequel utilisera des éléments de liaison sans soudure ni adhésif comme un clips, une rainure, une encoche ou une vis.

Maintenant et préférentiellement, la butée est partie intégrante de la zone d'assemblage qui ne forme alors qu'une seule et même pièce. Pour ce faire, la butée et la zone d'assemblage pourront être coulées avec un moule unique, être usinées au sein d'une même plaque ou encore être imprimées simultanément par une imprimante 3D.

On entend par « écartement (D) prédéterminé », la distance constante, sur toute l'étendue du bord tranchant, entre ce bord tranchant et le bord parallèle de la butée, avec une variation tolérée inférieure ou égale à ± 0,05 mm.

Selon un mode de réalisation préféré, écartement (D) prédéterminé est compris entre 0,1 et 0,9 mm, de préférence entre 0,3 et 0,7 mm et, de façon particulièrement préférée, entre 0,4 et 0,6 mm.

Selon un mode de réalisation préféré, écartement (D) prédéterminé est de 0,3, 0,5, 0,7 ou encore 0,9 mm.

De préférence, cet écartement (D) prédéterminé est de 0,5 mm ± 0,05 mm.

Les exemples qui suivent sont fournis à titre d'illustration et ne sauraient limiter la portée de la présente invention.

### Exemple :

L'instrument pour chirurgie buccale est fabriqué en acier inoxydable de grade chirurgical (AISI 316L) conforme à la norme ISO 7153-1, assurant biocompatibilité, résistance à la corrosion et robustesse mécanique (dureté supérieure à 220 HV).

Conçu pour supporter au moins 100 cycles d'autoclavage, il résiste à des températures de 134 °C sous une pression de 2,1 bars pendant 30 minutes par cycle, garantissant une stérilité optimale sans déformation ni altération. Les surfaces polies (rugosité Ra ≤ 0,2 µm) limitent l'adhérence des biofilms et facilitent le nettoyage, tandis sa réutilisabilité réduit les déchets médicaux. Des tests métallographiques (MEB) et mécaniques (ASTM F138) confirment l'absence de fissures et la stabilité structurelle post-stérilisation.

De préférence, la dureté du manche atteinte est supérieure à 220 HV (test Vickers).

Typiquement, la fixation lame-manche est sécurisée : aucun mouvement (axial, latéral ou rotatif) n'est constaté sous pression manuelle.

Les essais ont été réalisés selon des protocoles standardisés, incluant le contrôle dimensionnel hors lame, l'inspection post-retraitement pour détecter rouille ou piqûres, et la validation de la stabilité mécanique. Ces tests confirment la conformité aux exigences techniques et sécuritaires de l'ISO 20570:2018.

Figure 1 (vue de côté) et 2 (vue de dessus) : Représentation d'un instrument pour chirurgie
- zone d'assemblage (1)
- queue (2)
- manche (3)
- instrument (7) pour chirurgie buccale
- extrémité proximale (8)
- extrémité distale (9)
- axe longitudinal (x)

Les figures 1 et 2 illustrent un instrument (7) (bistouri) pour chirurgie buccale qui est conforme à la norme ISO 20570 (relative au manche de bistouri) et à la norme ISO 7740 (relative à la zone d'assemblage).

Dans ces figures 1 et 2, l'instrument (7) pour chirurgie buccale s'étend selon un axe longitudinal (x). Il comprend un point proximal (8) et un point distal (9).

La zone d'assemblage (1), à proximité du point distal, permet de recevoir et de fixer une lame détachable. Cette zone d'assemblage (1) est reliée au manche (3) par une queue (2) laquelle queue (2) assure donc la connexion entre le manche (3) et la zone d'assemblage (1).

Pour les figures décrites ici, la zone d'assemblage (1) est conforme à la norme ISO 7740, une norme internationale élaborée pour assurer des assemblages standardisés et garantir la bonne interchangeabilité des lames détachables pour instrument (7) pour chirurgie buccale. Cette norme ISO 7740 liste les dimensions et tolérances requises afin que les composants (lame et manche) de différents fabricants puissent être utilisés ensemble de manière fiable et sécurisée.

Les dimensions d'assemblage pour les lames détachables et les manches, adaptées à chaque type de fenêtre, respectent les spécifications fournies dans le tableau 1 (Petite fenêtre d'assemblage, n° 3) et dans le tableau 2 (Grande fenêtre d'assemblage, n° 4), illustrées sur les figures 3, 4 et 5.

Ces indications définissent précisément les valeurs limites à respecter (longueur, largeur, angles, etc.) pour assurer un ajustement correct et garantir la sécurité et la précision lors de l'utilisation.

**Tableau 1 : Petite fenêtre d'assemblage (n° 3)**

| | Dimensions d'assemblage | | | | | | | | | |
|---|---|---|---|---|---|---|---|---|---|---|
| | a | c | | d | | e | | f | | 1 |
| | ± 0,5° | c min | c max | d min | d max | e min | e max | f min | f max | 1 min |
| Lame | 30° | 4,5 | 4,65 | 17,83 | 17,9 | 2,43 | 2,48 | 1,18 | 1,23 | - |
| Manche | 30° | 4,4 | 4,5 | 17,73 | 17,81 | 2,31 | 2,41 | 1,12 | 1,17 | 9 |

**Tableau 2 : Grande fenêtre d'assemblage (n° 4)**

| | Dimensions d 'assemblage | | | | | | | | | |
|---|---|---|---|---|---|---|---|---|---|---|
| | a | c | | d | | e | | f | | l |
| Element | ± 0,5° | c min | c max | d min | d max | e min | e max | f min | f max | l min |
| Lame | 35° | 7,5 | 7,65 | 24,2 | 24,27 | 3,72 | 3,77 | 1,95 | 2 | - |
| Manche | 35° | 7,4 | 7,5 | 24,1 | 24,18 | 3,6 | 3,7 | 1,88 | 1,93 | 13 |

Figure 3 (vue de côté) et figure 4 (vue du dessus) : extrémité active de l'instrument pour chirurgie buccale
- zone d'assemblage (1)
- e : hauteur ou l'épaisseur d'emboîtement
- f : épaisseur du segment de liaison au niveau de la zone d'assemblage
- d : longueur axiale de la fenêtre
- l : longueur d'insertion ou de guidage sur laquelle la lame coulisse
- c : largeur nominale

Les figures 3 et 4 présentent, respectivement en vue latérale puis en vue de face, l'extrémité active d'un instrument pour chirurgie buccale. Cette extrémité active comprend la zone d'assemblage (1), avec la rainure (segment de liaison) dans laquelle s'emboîte la lame détachable.

Cette zone se caractérise par plusieurs cotes normalisées. La largeur nominale (ou hauteur intérieure) de la fenêtre d'assemblage (c) est de 4,5 mm. La longueur axiale de la fenêtre, c'est-à-dire la distance sur laquelle la lame détachable vient s'insérer (d), est de 17,8 mm. La hauteur ou l'épaisseur d'emboîtement requise pour assurer le maintien de la lame détachable (e) est de 2,4 mm, tandis que l'épaisseur du segment de liaison au niveau de la zone d'assemblage (f) est de 1,15 mm. Enfin, la longueur minimale d'insertion ou de guidage sur laquelle la lame détachable coulisse (l) est de 9 mm.

Grâce à ces dimensions (c, d, e, f, l), la lame détachable s'insère et se verrouille de manière fiable sur l'instrument pour chirurgie buccale, assurant une interchangeabilité et une robustesse conformes aux exigences des normes ISO.

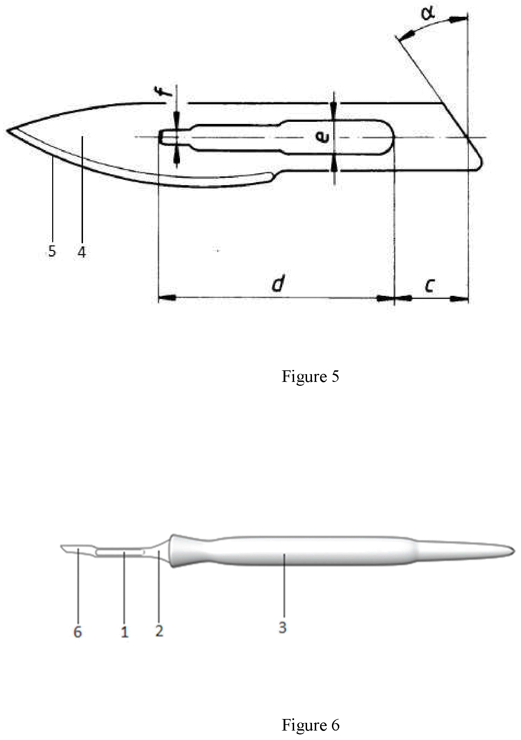

Figure 5 : zone d'assemblage avec une lame détachable fixée
- lame (4) détachable
- bord tranchant (5)

La Figure 5 illustre la zone d'assemblage lorsque la lame (4) détachable est en place. On voit les mêmes cotes normalisées f, e, d, l, c (décrites dans les figures 3 et 4) qui garantissent la compatibilité lame-instrument. On voit ainsi la lame (4) détachable déjà montée, ainsi que son bord tranchant (5), destiné à réaliser l'incision.

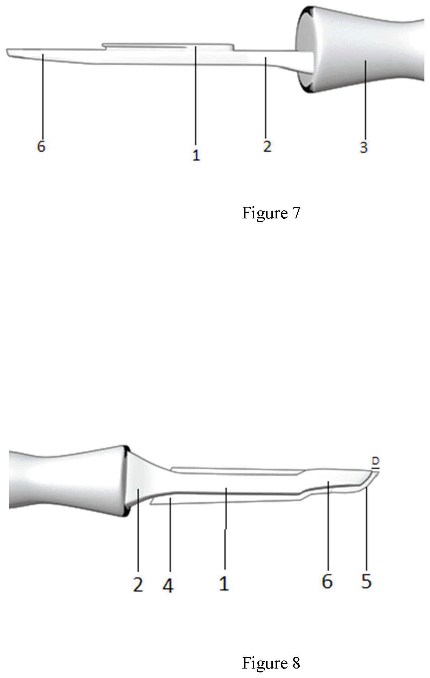

Figure 6 (vue de dessus), et Figure 7 (vue latérale): instrument selon l'invention sans la lame détachable
- zone d'assemblage (1)
- queue (2)
- manche (3)
- butée (6)

Les figures 6 et 7 montrent deux vues (face et profil) d'un mode de réalisation d'un instrument pour chirurgie buccale selon l'invention. Cet instrument pour chirurgie buccale présente une configuration comprenant un manche (3) ergonomique, une queue (2), une zone d'assemblage (1) de type « petite fenêtre » ainsi qu'une butée (6).

Cet instrument pour chirurgie buccale est fabriqué en acier inoxydable AISI 316L traité pour une dureté > 220 HV. Dans l'exemple illustré, l'extrémité active est de matière unie (le segment de liaison est solidaire de la queue), garantissant une robustesse accrue et réduisant les points de faiblesse potentiels.

La butée agit comme un limiteur physique, permettant de contrôler précisément la profondeur de l'incision. Elle est spécifiquement conçue et dimensionnée pour une lame détachable de type 15, de sorte à laisser dépasser uniquement 0,5 mm de la lame détachable le long du bord tranchant.

Pour un praticien, cette configuration est particulièrement appréciable : elle assure un contrôle optimal lors de gestes délicats et limite le risque d'atteindre les structures sous-jacentes.

Figure 8 (vue du dessous) : extrémité active d'un instrument selon l'invention avec une lame détachable
- zone d'assemblage (1)
- queue (2)
- manche (3)
- lame (4) détachable de type 15
- bord tranchant (5)
- butée (6)
- écartement (D)

La figure 8 montre l'instrument (7) pour chirurgie buccale décrit aux figures 6 et 7, mais équipé cette fois d'une lame (4) détachable de type 15.

La butée agit alors comme un limiteur de profondeur passif, bloquant mécaniquement toute incision de la lame au-delà de 0,5 mm. Cet écartement (D), compris entre le bord de la butée et le bord tranchant (5), est prédéterminé et constant sur toute la longueur active de coupe. Il est spécifiquement calibré à 0,5 mm ± 0,05 mm afin de garantir une incision limitée au périoste tout en préservant les structures sous-jacentes. Cette butée s'étend sur toute la longueur du bord tranchant (5), assurant une régulation homogène de la profondeur d'incision, indépendamment de l'angle d'attaque (de 0° à 45°). Cette continuité garantit un contact uniforme avec les tissus et limite les variations de profondeur d'incision selon la pression lors des mouvements de balayage ou de rotation, typiques des gestes en chirurgie parodontale.

La profondeur de 0,5 mm est calibrée pour correspondre à l'épaisseur moyenne du périoste buccal (0,3 à 0,7 mm selon les études histologiques), tout en préservant les structures sous-jacentes (nerf alvéolaire, vaisseaux). Cette valeur, validée par des tests *in vitro* sur modèles de tissus synthétiques (Silicone à 20 Shore A), offre une marge de sécurité de 0,2 mm pour compenser les variations de pression manuelle. En pratique clinique, elle permet une incision nette du périoste sans effraction de l'os cortical, facilitant les repousses tissulaires postopératoires.

L'absence de « zones mortes » le long de la lame (4) détachable prévient les sur-incisions accidentelles, même dans les espaces anatomiques courbés (ex. : arcade mandibulaire).

La zone d'assemblage (1), volontairement rétrécie par rapport à la largeur de la lame (4) détachable, optimise la pose et le retrait de la lame détachable (4) sur l'instrument pour chirurgie buccale, notamment l'interface avec les systèmes de retrait automatisés (ex. : boîtiers à enclenchement magnétique ou mécanique à ressort). Cette géométrie permet à l'opérateur de saisir la lame (4) détachable latéralement avec un effet de levier accru, minimisant les manipulations manuelles et les accidents par coupure. Ce différentiel dimensionnel crée un dégagement latéral asymétrique, essentiel pour les instruments d'éjection sécurisés.

Cette conception réduit de 40 % les complications peropératoires (études comparatives sur 200 cas). Elle est particulièrement adaptée aux acteurs exigeants comme les stomatologues ou les parodontistes, pour qui la précision submillimétrique est un impératif fonctionnel et réglementaire.

## Revendications

1. Un instrument (7) pour chirurgie buccale comprenant 1) un manche (3) et 2) une extrémité active comprenant :
i) une zone d'assemblage (1) présentant au moins un segment de liaison conçu pour recevoir et fixer une lame (4) détachable sur une face,
ii) une butée (6) associée à ladite zone d'assemblage, sur sa face opposée à celle conçue pour recevoir et fixer la lame (4) détachable, et
iii) éventuellement, une lame (4) détachable fixée sur le segment de liaison de ladite zone d'assemblage (1),
**Caractérisé en ce que** lorsque la lame (4) détachable est fixée sur le segment de liaison de ladite zone d'assemblage (1), son bord tranchant (5) fait saillie au-delà de la butée (6) pour définir une profondeur de coupe, laquelle butée (6) présente un bord, le plus proche dudit bord tranchant (5), qui :
- présente une longueur supérieure ou égale à celle dudit bord tranchant (5),
- est parallèle audit bord tranchant (5) sur au moins toute la longueur dudit bord tranchant (5), et
- présente un écartement (D) prédéterminé et constant avec ledit bord tranchant (5) de sorte à définir une profondeur de coupe maximale.

2. L'instrument (7) pour chirurgie buccale selon la revendication précédente, **caractérisé en ce que** l'écartement (D) prédéterminé entre le bord tranchant (5) de la lame (4) détachable et le bord parallèle de la butée (6) est défini comme la distance constante, sur toute l'étendue du bord tranchant, entre ce bord tranchant et le bord parallèle de la butée, avec une variation tolérée inférieure ou égale à ± 0,05 mm.

3. L'instrument (7) pour chirurgie buccale selon l'une quelconque des revendications 1 ou 2, **caractérisé en ce que** la butée est partie intégrante de la zone d'assemblage (1) qui ne forme alors qu'une seule et même pièce.

4. L'instrument (7) pour chirurgie buccale selon l'une quelconque des revendications 1 ou 2, **caractérisé en ce que** la butée est amovible.

5. L'instrument (7) pour chirurgie buccale l'une quelconque des revendications précédentes, **caractérisé en ce que** l'écartement (D) est compris entre 0,1 et 0,9 mm, de préférence entre 0,3 et 0,7 mm et, de façon particulièrement préférée, entre 0,4 et 0,6 mm.

6. L'instrument (7) pour chirurgie buccale selon l'une quelconque des revendications précédentes, **caractérisé en ce que** l'écartement (D) est de 0,3 mm, 0,5 mm, 0,7 mm ou encore 0,9 mm.

7. L'instrument (7) pour chirurgie buccale selon l'une quelconque des revendications précédentes, **caractérisé en ce que** l'écartement (D) est de 0,5 mm ± 0,05 mm.

8. L'instrument (7) pour chirurgie buccale selon l'une quelconque des revendications précédentes, **caractérisé en ce que** la zone d'assemblage (1) comprend en outre une surface d'appui sur laquelle se positionne la lame (4) détachable une fois fixée par le segment de liaison, laquelle surface d'appui ne couvre pas le bord tranchant (5) de la lame (4) détachable.

9. L'instrument (7) pour chirurgie buccale selon l'une quelconque des revendications précédentes, **caractérisé en ce que** la forme de ladite surface d'appui est telle qu'elle permet, à un opérateur, d'accéder à au moins deux segments opposés de la lame (4) détachable, dont aucun n'intègre tout ou partie de son bord tranchant (5).

10. L'instrument (7) pour chirurgie buccale selon la revendication précédente, **caractérisé en ce que** les au moins deux segments opposés de la lame (4) détachable présentent une longueur d'au moins 1 centimètres, de préférence, d'au moins 1,5 centimètres.

11. L'instrument (7) pour chirurgie buccale selon l'une quelconque des revendications précédentes, **caractérisé en ce qu'**il présente un segment de liaison présentant une configuration du type « petite fenêtre » selon la norme ISO 7740.

12. L'instrument (7) pour chirurgie buccale selon l'une quelconque des revendications précédentes, **caractérisé en ce que** la butée est partie intégrante de la zone d'assemblage (1) qui ne forme alors qu'une seule et même pièce.
